(19) Europäisches Patentamt
European Patent Office
Office européen des brevets

(11) EP 2 253 310 A1

(12) EUROPEAN PATENT APPLICATION

(43) Date of publication:
24.11.2010 Bulletin 2010/47

(51) Int Cl.:
A61K 9/22 (2006.01)  A61K 9/36 (2006.01)
A61K 31/137 (2006.01)

(21) Application number: 10173302.0

(22) Date of filing: 01.06.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(30) Priority: 01.06.2006 US 810021 P

(62) Document number(s) of the earlier application(s) in accordance with Art. 76 EPC:
07777371.1 / 2 020 994

(71) Applicant: Schering-Plough Healthcare Products, Inc.
Memphis, TN 38151 (US)

(72) Inventors:
• Patton, John W.
Germantown, TN 38138 (US)
• Kabir, Mohammed
Lakeland, TN 38002 (US)
• Reo, Joseph P.
Lakeland, TN 38002 (US)

(74) Representative: UEXKÜLL & STOLBERG
Patentanwälte
Beselerstrasse 4
22607 Hamburg (DE)

Remarks:
This application was filed on 18-08-2010 as a divisional application to the application mentioned under INID code 62.

(54) **Sustained release pharmaceutical formulation comprising phenylephrine**

(57) Pharmaceutical compositions comprising phenylephrine in a sustained release oral dosage formulation. Compositions can comprise phenylephrine alone or phenylephrine in combination additional pharmaceutically active substances such as an antihistamine and/or an analgesic.

FIGURE 1
Dissolution Profile of a Tablet

**Description**

**FIELD OF THE INVENTION**

This invention relates to a sustained release pharmaceutical dosage form containing the decongestant phenylephrine.

**BACKGROUND OF THE INVENTION**

Phenylephrine and its pharmaceutically acceptable salts are recognized as safe and effective nasal decongestants. Commercially available formulations include nasal jelly, nasal drops, and nasal spray (i.e. Alconefrin® Nasal Drops or Neo-Synephrine® Nasal Jelly) as well as immediate release oral tablets or gelatin capsules (i.e. Sudafed PE™ or DayQuil® LiquiCaps). Phenylephrine or a pharmaceutically acceptable salts as currently formulated is commonly administered every four hours for the relief of nasal congestion due to the short half-life of phenylephrine. Thus, there is a need for sustained release formulations of phenylephrine that can be administered less frequently, for example, once every eight hours, every twelve hours or possibly every twenty four hours.

Sustained release formulations result in a decrease in the frequency of drug administration thereby improving patient compliance. In addition, sustained drug release systems produce constant therapeutic plasma levels of active ingredients as compared to fluctuations seen when multiple doses of a conventional formulation are given. Sustained drug release may decrease the severity and frequency of side effects from multiple dosages or from pulsed release systems.

U.S. Pat. No. 4,792,452 discloses a tablet formulation composed of up to about 45% by weight of a pH-dependent salt of alginic acid, up to about 35% by weight of a pH-independent hydrocolloid gelling agent, binder and excipients. Release of the drug is therefore affected by the varying pH of the gastrointestinal tract. Australian patent application AU-B-56761/86 discloses examples of sustained release formulations for aspirin and theophylline including specific hydroxypropylmethylcelluloses. AU-B-56761/86 also describes phenylephrine as one of at least twenty-seven drugs or types of drugs that are typical moisture sensitive drugs. JP 2005-60294 discloses stabilized compositions of phenylephrine containing carbinoxamine and various salts.

A decongestant is commonly administered orally in combination with an antihistamine for relieving nasal congestion associated with allergic rhinitis. Antihistamines with long half lives compared to phenylephrine are known. When the decongestant is phenylephrine or its pharmaceutically acceptable salt, and the antihistamine is a long-acting antihistamine, then the dosage form should preferably be designed such that the long acting antihistamine is released in a conventional manner and phenylephrine is released at a sustained rate such that the pharmaceutical composition is suitable for eight hour or longer administration.

Loratadine is disclosed in U.S. Pat. No. 4,282,233 as a non-sedating antihistamine useful, for example, in alleviation of seasonal allergic rhinitis symptoms such as sneezing and itching, and in the treatment of chronic idiopathic urticaria in patients six years or older. Loratadine is available in the form of conventional tablets that release loratadine in a conventional manner by the processes of disintegration and dissolution such that loratadine begins to elicit its antihistaminic effect within 1 to 3 hrs and the effect lasts in excess of 24 hrs. The tablets are thus orally administered only once daily. Azatadine is disclosed in Belgian Patent No. 647,043 and in corresponding U.S. Pat. Nos. 3,326,924 and 3,419,565. The elimination half-life is reported to be 9-12 hrs. Terfenadine and fexofenadine are disclosed in U.S. Pat. No. 3,878,217 and have a duration of action of 12 to 24 hrs, and greater than 24 hrs., respectively. Cetirizine disclosed in U.S. Pat. No. 4,525,358; astemizole in U.S. Pat. No. 4,219,559, and levocabastine in U.S. Pat. No. 4,369,184; have a duration of action of 12 to 24 hrs, greater than 24 hrs, and 16 to 24 hrs; respectively.

It is an object of the present invention to provide an oral sustained release pharmaceutical composition that releases phenylephrine or a pharmaceutically acceptable salt thereof over a period of at least eight hours, preferably at least 12 hours, more preferably at least 24 hours.

It is another object of the invention to provide a pharmaceutical composition that provides for combined administration in a single dose form of a therapeutically effective amount of phenylephrine or pharmaceutically acceptable salt thereof and an additional therapeutic composition, such as an antihistamine, that has a natural half-life longer than phenylephrine. These and other objectives of the invention are satisfied by the invention described and claimed below, which provides a single dose of phenylephrine to achieve a distribution over at least eight hours. As described in the examples below, one embodiment of the invention has been demonstrated to provide a single dose of phenylephrine that is bioequivalent to two doses of immediate release phenylephrine given at four hour intervals. Additionally, formulations prepared according to the invention are stable, with a shelf life of at least two years. Formulations according to the invention may comprise phenylephrine alone or phenylephrine in combination with additional pharmaceutically active agents, including antihistamines, analgesics, and others.

**SUMMARY OF THE INVENTION**

Thus, the invention provides a pharmaceutical composition in the form of a solid dosage for oral administration, the composition comprising a core comprising phenylephrine or a pharmaceutically acceptable salt thereof in a sustained release matrix, and further comprising one or more hydrophilic or hydrophobic polymers outside the core.

The invention further provides a pharmaceutical composition formulated for sustained release of therapeutically effective phenylephrine dose for at least eight hours after a single dose is administered to a human subject, the composition comprising phenylephrine within a polymer matrix comprising hydroxypropyl methylcellulose, wherein the composition exhibits a mean AUC and/or $C_{max}$ in the human subject equivalent to 50% to 125% of the AUC and/or $C_{max}$ obtained when two doses of a standard immediate release formulation comprising 10 mg of phenylephrine is administered. In a preferred embodiment, the composition exhibits a mean AUC and/or $C_{max}$ in the human subject equivalent to 80% to 125% of the AUC and/or $C_{max}$ obtained when two doses of a standard immediate release formulation comprising 10 mg of phenylephrine is administered.

The invention further provides a pharmaceutical composition formulated for sustained release of therapeutically effective phenylephrine dose for at least twelve hours after a single dose is administered to a human subject, the composition comprising phenylephrine within a polymer matrix comprising hydroxypropyl methylcellulose, wherein the composition exhibits a mean AUC and/or $C_{max}$ in the human subject equivalent to 50% to 125% of the AUC and/or $C_{max}$ obtained when three doses of a standard immediate release formulation comprising 10 mg of phenylephrine is administered. In a preferred embodiment, the composition exhibits a mean AUC and/or $C_{max}$ in the human subject equivalent to 80% to 125% of the AUC and/or $C_{max}$ obtained when three doses of a standard immediate release formulation comprising 10 mg of phenylephrine is administered.

The invention also provides a pharmaceutical composition formulated for sustained release of therapeutically effective phenylephrine dose for at least twenty four hours after a single dose is administered to a human subject, the composition comprising phenylephrine within a polymer matrix comprising hydroxypropyl methylcellulose, wherein the composition exhibits a mean AUC and/or $C_{max}$ in the human subject equivalent to 50% to 125% of the AUC and/or $C_{max}$ obtained when six doses of a standard immediate release formulation comprising 10 mg of phenylephrine is administered. In a preferred embodiment, the composition exhibits a mean AUC and/or $C_{max}$ in the human subject equivalent to 80% to 125% of the AUC and/or $C_{max}$ obtained when six doses of a standard immediate release formulation comprising 10 mg of phenylephrine is administered.

The invention also provides a pharmaceutical composition comprising phenylephrine or a pharmaceutically acceptable salt thereof in a sustained release polymer matrix, wherein said composition when administered once to a subject exhibits a mean AUC and/or $C_{max}$ which is at least 50% of the mean AUC and/or $C_{max}$ exhibited by two doses of a pharmaceutical composition containing 10 mg of phenylephrine and no sustained release polymer matrix. In a preferred embodiment, the composition when administered once to a subject exhibits a mean AUC and/or $C_{max}$ which is at least 80% of the mean AUC and/or $C_{max}$ exhibited by two doses of a pharmaceutical composition containing 10 mg of phenylephrine and no sustained release polymer matrix.

The invention further provides a pharmaceutical composition comprising phenylephrine or a pharmaceutically acceptable salt thereof in a sustained release polymer matrix, wherein said composition when administered once to a subject exhibits a mean AUC and/or $C_{max}$ which is at least 50% of the mean AUC and/or $C_{max}$ exhibited by three doses of a pharmaceutical composition containing 10 mg of phenylephrine and no sustained release polymer matrix. In a preferred embodiment, the composition when administered once to a subject exhibits a mean AUC and/or $C_{max}$ which is at least 80% of the mean AUC and/or $C_{max}$ exhibited by three doses of a pharmaceutical composition containing 10 mg of phenylephrine and no sustained release polymer matrix.

The invention also provides a pharmaceutical composition comprising phenylephrine or a pharmaceutically acceptable salt thereof in a sustained release polymer matrix, wherein said composition when administered once to a subject exhibits a mean AUC and/or $C_{max}$ which is at least 50% of the mean AUC and/or $C_{max}$ exhibited by six doses of a pharmaceutical composition containing 10 mg of phenylephrine and no sustained release polymer matrix. In a preferred embodiment, the composition when administered once to a subject exhibits a mean AUC and/or $C_{max}$ which is at least 80% of the mean AUC and/or $C_{max}$ exhibited by six doses of a pharmaceutical composition containing 10 mg of phenylephrine and no sustained release polymer matrix.

The invention further provides a pharmaceutical composition comprising phenylephrine or a pharmaceutically acceptable salt thereof, wherein the composition exhibits an sustained release of phenylephrine from the composition when contacted with USP simulated intestinal fluid, such that no more than 30 percent of the phenylephrine or pharmaceutically acceptable salt thereof is released from the pharmaceutical composition within one hour, no more than 60 percent of the phenylephrine or pharmaceutically acceptable salt thereof is released from the pharmaceutical composition within four hours, no more than 90 percent of the phenylephrine or pharmaceutically acceptable salt thereof is released from the pharmaceutical composition within about 8 hours, and at least 99 percent of the phenylephrine or pharmaceutically acceptable salt thereof is released from the pharmaceutical composition within about 24 hours.

**BRIEF DESCRIPTION OF THE DRAWINGS**

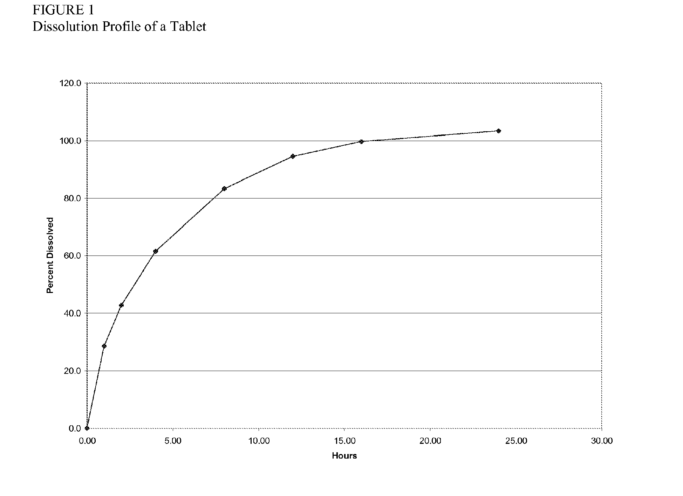

Figure 1: Release profile of phenylephrine HCl from a tablet was over a 24 hour time period. The dissolution study was conducted with USP simulated intestinal fluid using an USP Apparatus II stirring set at 50 rpm. At each time interval, a sample of the solution was analyzed to determine the percent of phenylephrine HCl dissolved.

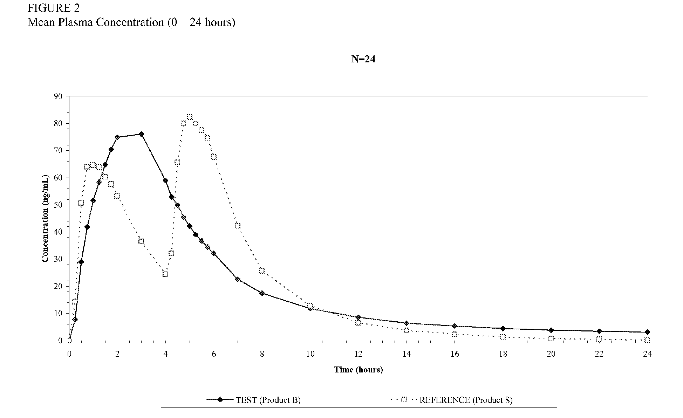

Figure 2: Mean plasma concentration over 24 hours from a bioequivalence study comparing a single dose of 30mg phenylephrine in a tablet according to the invention (Test Product B) to two 10mg phenylephrine immediate release tablets (Reference Product S) dosed four hours apart.

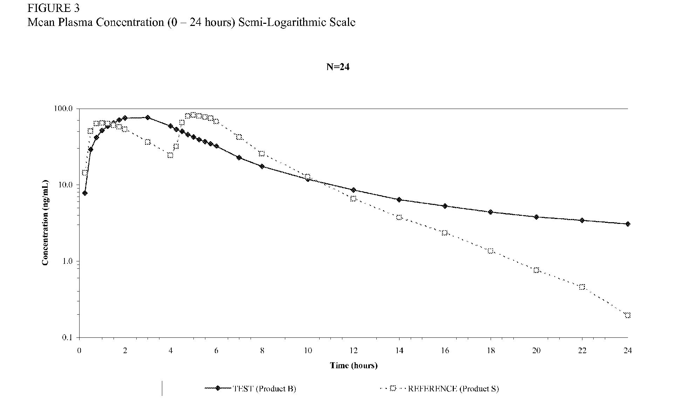

Figure 3: Mean plasma concentration over 24 hours on a semi-logarithmic scale from a bioequivalence study comparing a single does of 30mg phenylephrine in a tablet according to the invention (Test Product B) to two 10mg phenylephrine immediate release tablets (Reference Product S) dosed four hours apart.

**DETAILED DESCRIPTION OF THE INVENTION**

The present invention provides a pharmaceutical composition in the form of a solid dosage comprising phenylephrine or a pharmaceutically acceptable salt and one or more sustained release polymers together with excipients to provide a composition that releases phenylephrine over a period of about eight or more hours. The composition according to the invention is bioequivalent to at least two standard release dosage forms given four hours apart when measured by $C_{max}$ and AUC for serum analysis in human subjects. In a preferred embodiment, the invention is bioequivalent to at least three standard release dosage forms given four hours apart when measured by $C_{max}$ and AUC for serum analysis in human subjects. In a further preferred embodiment, the composition according to the invention is bioequivalent to at least six standard release dosage forms given four hours apart when measured by $C_{max}$ and AUC for serum analysis in human subjects.

As used herein in relation to a preferred embodiment, the term bioequivalent is used according to its commonly understood meaning, i.e., a composition exhibiting between 80% and 125% of the $C_{max}$ and AUC for serum analysis in human subjects of a standard release dosage form.

According to one embodiment of the invention, an amount of phenylephrine is formulated for sustained release within a tablet core. As used herein, the term sustained release refers to release of the active agent from the pharmaceutical composition so that it becomes available for bio-absorption in the subject, primarily in the gastrointestinal tract of the subject, over a prolonged period of time, such as about 1 hour to 24 hours or more. In certain embodiments of the composition of the invention, that period of time will be at least about 8 hours, at least about 12 hours, or at least about 24 hours. The term sustained release also encompasses what is otherwise referred to as extended release, controlled release, or sustained delivery. The release rate of the active agent is primarily controlled by dissolution of the active agent in gastrointestinal fluid and subsequent diffusion out of the tablet independent of pH, but can also be influenced by physical processes of disintegration and erosion of the tablet. Due to the relatively short half life of phenylephrine, therapeutic blood serum concentrations of phenylephrine are primarily a result of release of phenylephrine from the tablet over a prolonged period of time. Formulations according to the invention provide a single dose of phenylephrine to achieve a therapeutic blood serum concentration of phenylephrine in a subject in need thereof for an extended period of time so as to provide for a therapeutic effect of phenylephrine in the subject. In separately preferred embodiments of this invention, phenylephrine is released from the tablet to result in a therapeutic blood serum concentration for at least 8 hours, or at least 12 hours or at least 24 hours from a single dose. The release rate from the tablet is independent of pH, but is highly dependent on the solubility profile for phenylephrine. Active agents other than phenylephrine have different release rates than phenylephrine, and therefore are not predictive for compositions according to the invention.

In a preferred embodiment, phenylephrine is used in combination with an additional active ingredient. When phenylephrine is used in combination with an antihistamine, the antihistamines can be of $H_1$ or $H_2$ antagonists or other types of histamine release inhibitors. The $H_1$ antagonists can be sedating or non-sedating, such as diphenhydramine, chlorpheniramine, tripelennamine, promethazine, clemastine, doxylamine, astemizole, terfenadine, and loratadine, among others. Examples of $H_2$ antagonists include, but are not limited to, cimetadine, famotidine, nizatidine, and ranitidine. Examples of histamine-release-inhibitors include, but are not limited to, cromolyn.

If the optional active ingredient(s) used has similar water solubility to phenylephrine, it can located within the core of the tablet, i.e., within the polymer matrix for controlled release. In additional embodiments, the optional active ingredient(s) has a long half-life compared to phenylephrine and does not require controlled release, but rather is released at a rapid or immediate release while phenylephrine is released at a sustained rate. In such cases, the optional active ingredient(s) can be located outside the core of the tablet.

The core of the tablet can also include additional inactive pharmaceutically acceptable carrier material. The term pharmaceutically acceptable carrier refers to any non-toxic solid, semisolid or liquid filler, diluent, encapsulating material or formulation auxiliary of any type. Suitable pharmaceutical carriers are described in Gennaro et al., (1995) Remington's Pharmaceutical Sciences, Mack Publishing Company. In a preferred embodiment the carrier material comprises solid pharmaceutical excipients such as starch, cellulose, talc, glucose, lactose, sucrose, gelatin, malt, rice, flour, chalk, silica gel, magnesium stearate, sodium stearate, glycerol monostearate, sodium chloride, dried skim milk and the like. In a preferred embodiment, the core of the pharmaceutical composition of the invention further comprises lactose, which may be in any of its pharmaceutically acceptable grades, including for example, β-lactose, anhydrous α-lactose, α-lactose monohydrate. In certain instances it may be important to control the water content of the materials used in the formulation of the pharmaceutical composition, for example to provide long term stability, for example for at least one, two, three or more years, and/or to control microbial growth during long term storage. In those instances, anhydrous forms of carrier materials may be preferred.

The sustained release polymer comprising the core of the tablet is hydroxypropyl methylcellulose. The sustained release polymer may be present in an amount from about 15% to 50%, preferably from about 20% to 40%, more preferably from about 25% to about 35% and even more preferably from about 29% to 31 % by weight of the tablet. According to one embodiment of the invention, the sustained release polymer comprises hydroxypropyl methylcellulose in an amount of about 30% by total weight of the tablet. In a preferred embodiment, the sustained release polymers are combined with lactose as a major component of the tablet. For example, for a tablet comprising about 30% by weight hydroxypropyl methylcellulose and about 4 % by weight hydroxypropylcellulose, about 59.5% by weight of the tablet may be lactose.

Examples of hydroxypropyl methylcellulose polymers that may be used in the present invention include those available from Dow Chemical Co. (Michigan, USA) under the brand name Methocel, such as, Methocel K100M CR, Methocel K3, Methocel K15M, and the like. Hydroxypropyl methylcellulose is also known as hypromellose.

As an example of a binder material, hydroxypropylcellulose polymers may be used in the present invention including, for example, those available under the brand names Klucel™ from Aqualon (Delaware, USA) such as Klucel EXF™, Klucel JF™, Klucel HF™, and Nisso HPC™ from Nippon Soda Co., Ltd. (Tokyo, Japan), such as HPC-LT™, HPC-M™, and the like. Hydroxypropylcellulose can be present in the compositions of the invention in an amount up to about 10%, preferably up to about 7.5%, more preferably up to about 4% of the total weight of the composition. Additional cellulose ethers may be present in the tablet, outside the core, in addition to hydroxypropylcellulose, such as other water soluble or swellable polymers including sodium carboxymethyl cellulose, xanthan gum, acacia, tragacanth gum, guar gum, karaya gum, alginates, gelatin, albumin and the like.

The dosage forms according to the invention are solid, and may take any customary form for oral administration, such as a tablet, a pill, a capsule, and the like. A preferred example of the solid dosage form is a compressed tablet. Dosage forms according to the invention may further contain standard excipients, such as disintegrants, glidants, binding agents, and antiadherents.

A sustained release formulation of the present invention may further comprise pharmaceutical additives including, but not limited to: lubricants such as magnesium stearate, calcium stearate, zinc stearate, powdered stearic acid, hydrogenated vegetable oils, talc, polyethylene glycol, and mineral oil; colorants such as various FD&C colors; binders such as sucrose, lactose, gelatin, starch paste, acacia, tragacanth, povidone, polyethylene glycol, pullulan and corn syrup; glidants such as colloidal silicon dioxide and talc; surface active agents such as sodium lauryl sulfate, dioctyl sodium sulfosuccinate, triethanolamine, polyoxyethylene sorbitan, poloxalkol, and quarternary ammonium salts; preservatives and stabilizers; excipients such as lactose, mannitol, glucose, fructose, xylose, galactose, sucrose, maltose, xylitol, sorbitol, chloride, sulfate and phosphate salts of potassium, sodium, and magnesium; and/or any other pharmaceutical additives known to those of skill in the art such as microcrystalline cellulose or dicalcium phosphate. Standard tablet excipients may be included in formulations according to the invention.

Optionally, the blend of phenylephrine, sustained release polymer, and excipients may be converted into granules by conventional means. For example, a wet granulation process including alcohol may be used. In one embodiment, tablets are manufactured by dry blending and granulating of the active ingredient with excipients, addition of anti-oxidant agents, chelating agents, moisture scavenging agents, or other stabilization aids such as magnesium stearate, and control of moisture levels in the granulated/compressed product to prevent phenylephrine degradation, use of a coated phenylephrine salt or use of a combination manufacturing process which separates phenylephrine salt from a wet granulation process and incorporates the active ingredient into the process by dry granulation or mixing. Direct compression and other conventional methods may be used to form tablets. Optionally, the blend or the granulated blend may be compressed into a core and coated with a polymeric film. Stability and degradation analyses can be performed according to International Conference on Harmonization (ICH) standards as described in "Impurities in New Drug Products" guidelines to simulate two or more years of shelf life. For example, stability testing can be performed at 40 degrees Celsius / 75% relative humidity for a 3-month time period. Standard pharmaceutical storage conditions are known in the art. Compositions according to the invention can be assayed to meet all ICH guidelines for active pharmaceutical assay with degradant levels which are below reporting limits, preferably below identification limits, most preferably below qualification limits.

The invention further provides a pharmaceutical composition comprising phenylephrine or a pharmaceutically acceptable salt thereof, wherein the composition exhibits an sustained release of phenylephrine from the composition when contacted with USP simulated intestinal fluid. In this aspect of the invention, the pharmaceutical composition is formulated such that when tested in in vitro methods that simulate in vivo conditions, the pharmaceutical composition can be analyzed to demonstrate a sustained release of phenylephrine from the composition. In one embodiment, the pharmaceutical composition will provide a sustained release for a period of at least 8 hours such that a determination can be made that less than the total amount of phenylephrine has been dissolved in the USP simulated intestinal fluid over that time period. In additional embodiments, the time period is at least 12 hours, at least 16 hours, at least 20 hours or at least 24 hours.

In a preferred embodiment the composition exhibits sustained release of phenylephrine such that no more than 30 percent of the phenylephrine or pharmaceutically acceptable salt thereof is released from the pharmaceutical composition within one hour of contact with the USP simulated intestinal fluid, no more than 60 percent of the phenylephrine or pharmaceutically acceptable salt thereof is released from the pharmaceutical composition within four hours, no more than 90 percent of the phenylephrine or pharmaceutically acceptable salt thereof is released from the pharmaceutical composition within about 8 hours, and at least 99 percent of the phenylephrine or pharmaceutically acceptable salt thereof is released from the pharmaceutical composition within about 24 hours.

The subject to whom the composition according to the invention is to be administered is not restricted. The dosage varies depending on the size and age of the patient, the severity of the symptoms, and the like. The administration is preferably carried out by adjusting the dosage based on various factors taken into account by those of ordinary skill in the art, which include the subject's age, weight, prior dose response, and is preferably administered once or twice daily.

In the following, further embodiments of the invention are described by numbered paragraphs:

1. A pharmaceutical composition in the form of a solid dosage for oral administration, the composition comprising a core comprising phenylephrine or a pharmaceutically acceptable salt thereof in a sustained release matrix and further comprising one or more hydrophilic or hydrophobic polymers outside the core.

2. The composition of paragraph 1, wherein the sustained release matrix comprises hydroxypropyl methylcellulose.

3. The composition according to paragraph 1, wherein the core displays increased resistance to disintegration and erosion in the gastrointestinal tract to allow for sustained release of the phenylephrine or a pharmaceutically acceptable salt thereof for at least an 8 hour time period.

4. The composition according to paragraph 1, wherein the core displays increased resistance to disintegration and erosion in the gastrointestinal tract for sustained release of the phenylephrine or a pharmaceutically acceptable salt thereof for at least a 12 hour time period.

5. The composition according to paragraph 1, wherein the core displays increased resistance to disintegration and erosion in the gastrointestinal tract for sustained release of the phenylephrine or a pharmaceutically acceptable salt thereof for at least a 24 hour time period.

6. The composition according to paragraph 1, wherein the hydrophilic or hydrophobic polymers are selected from the group consisting of water soluble cellulosic ethers, polyethylene oxide, and mixtures thereof.

7. The composition according to paragraph 1, further comprising one or more pharmaceutically acceptable excipients.

8. The composition of paragraph 5, wherein the water soluble cellulosic ether is selected from the group consisting of methylcellulose, hydroxypropylcellulose, hydroxymethylcellulose, hydroxypropylmethylcellulose, hydroxyethylcellulose, carboxymethylcellulose, carboxyethylcellulose and mixtures thereof.

9. The composition of paragraph 1, wherein the hydrophobic polymer is crosslinked acrylic acid.

10. The composition of paragraph 1, wherein the phenylephrine or a pharmaceutically acceptable salt thereof comprises approximately 1 to 25% of the tablet weight, and the hydroxypropyl methylcellulose comprises approximately 10 to 50% of the tablet weight.

11. The composition of paragraph 1, wherein the composition is stable under standard pharmaceutical storage conditions for at least two years.

12. A pharmaceutical composition formulated for sustained release of therapeutically effective phenylephrine dose for at least eight hours after a single dose is administered to a human subject, the composition comprising phenylephrine within a polymer matrix, wherein the composition exhibits a mean AUC and/or $C_{max}$ in the human subject equivalent to 80% to 125% of the AUC and/or $C_{max}$ obtained when two doses of a standard immediate release formulation comprising 10 mg of phenylephrine is administered.

13. A pharmaceutical composition formulated for sustained release of therapeutically effective phenylephrine dose for at least twelve hours after a single dose is administered to a human subject, the composition comprising phenylephrine within a sustained release polymer matrix, wherein the composition exhibits a mean AUC and/or $C_{max}$ in the human subject equivalent to 80% to 125% of the AUC and/or $C_{max}$ obtained when three doses of a standard immediate release formulation comprising 10 mg of phenylephrine is administered.

14. A pharmaceutical composition formulated for sustained release of therapeutically effective phenylephrine dose for at least twenty four hours after a single dose is administered to a human subject, the composition comprising phenylephrine within a polymer matrix, wherein the composition exhibits a mean AUC and/or $C_{max}$ in the human subject equivalent to 80% to 125% of the AUC and/or $C_{max}$ obtained when six doses of a standard immediate release formulation comprising 10 mg of phenylephrine is administered.

15. A pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by two doses of a pharmaceutical composition containing 10 mg of phenylephrine and no sustained release polymer matrix.

16. A pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by three doses of a pharmaceutical composition containing 10 mg of phenylephrine and no sustained release polymer matrix.

17. A pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by six doses of a pharmaceutical composition containing 10 mg of phenylephrine and no sustained release polymer matrix.

18. The pharmaceutical composition of any of paragraphs 12-17 wherein the sustained release polymer matrix comprises hydroxypropyl methylcellulose.

19. The composition of paragraph 1 comprising an additional therapeutic agent.

20. The composition of paragraph 19 wherein the additional therapeutic agent is located outside the core.

21. The composition of paragraph 18 wherein the additional therapeutic agent is chosen from the group consisting of an antihistamine, an analgesic and combinations thereof.

22. The composition of paragraph 19 wherein the antihistamine is chosen from the group consisting of diphenhydramine, chlorpheniramine, tripelennamine, promethazine, clemastine, doxylamine, astemizole, terfenadine, loratadine, cimetadine, famotidine, nizatidine, ranitidine, cromolyn and combinations thereof.

23. A pharmaceutical composition comprising phenylephrine or a pharmaceutically acceptable salt thereof, wherein the composition exhibits an sustained release of phenylephrine from the composition when contacted with USP simulated intestinal fluid.

24. The pharmaceutical composition of paragraph 23 that provides a sustained release for a period of at least 8 hours such that a determination can be made that less than the total amount of phenylephrine has been dissolved in the USP simulated intestinal fluid over said time period.

25. A pharmaceutical composition comprising phenylephrine or a pharmaceutically acceptable salt thereof, wherein the composition exhibits an sustained release of phenylephrine from the composition when contacted with USP simulated intestinal fluid, such that no more than 30 percent of the phenylephrine or pharmaceutically acceptable salt thereof is released from the pharmaceutical composition within one hour, no more than 60 percent of the phenylephrine or pharmaceutically acceptable salt thereof is released from the pharmaceutical composition within four hours, no more than 90 percent of the phenylephrine or pharmaceutically acceptable salt thereof is released from the pharmaceutical composition within about 8 hours, and at least 99 percent of the phenylephrine or pharmaceutically acceptable salt thereof is released from the pharmaceutical composition within about 24 hours.

26. The composition of paragraph 25 comprising an additional therapeutic agent.

27. The composition of paragraph 26 wherein the additional therapeutic agent is chosen from the group consisting of an antihistamine, an analgesic and combinations thereof.

28. The composition of paragraph 27 wherein the antihistamine is chosen from the group consisting of diphenhydramine, chlorpheniramine, tripelennamine, promethazine, clemastine, doxylamine, astemizole, terfenadine, loratadine, cimetadine, famotidine, nizatidine, ranitidine, cromolyn and combinations thereof.

**EXAMPLES**

The present invention is illustrated by the following examples. Alternatives will be apparent to those skilled in the art and are considered to be within the scope of the invention, and therefore the examples are not to be construed to restrict the invention.

**Example 1: Tablet formulation.**

A tablet was made with the following components:

| | |
|---|---|
| lactose monohydrate | 297.5 mg |
| Methocel K100M CR[1] | 150.0 mg |
| phenylephrine HCl | 30.0 mg |
| Klucel EXF[2] | 20.0 mg |
| magnesium stearate | 2.5 mg |
| | total: 500.0 mg |

[1]: hydroxypropyl methylcellulose (hypromellose) 2208, 19-24% methoxyl content, 7-12% hydroxypropyl content.
[2]: hydroxypropylcellulose.

Phenylephrine HCl was passed through a 30 mesh screen. Lactose monohydrate, Methocel K100M CR, phenylephrine HCl, and Klucel EXF were charged into a blender and blended until uniform. A portion of half of the magnesium stearate was passed through a 30 mesh screen and added to blender with uniform mixture, and blended for an additional 1-3 minutes. The resulting mixture was granulated with a roller/compactor, sized with a mill, and blended for an additional 1-3 minutes. The remaining portion of magnesium stearate was passed through a 30 mesh screen and blended with the mixture for 1-3 minutes. The mixture was compressed into tablets. In an alternate procedure, the entire portion of magnesium stearate is passed through a 30 mesh screen and charged to the mixture in the blender in a single step. The mixture is blended for 1-3 minutes and the resulting granules are compressed into a tablet.

**Example 2: Tablet dissolution profile.**

The release profile of phenylephrine HCl from a tablet according to Example 1 was studied over a 24 hour time period. The dissolution study was conducted with USP simulated intestinal fluid using an USP Apparatus II stirring set at 50 rpm. At each time interval, a sample of the solution was analyzed to determine the percent of Phenylephrine HCl dissolved. Figure 1 presents the data graphically.

### Table 1 - Dissolution Profile

| Time (Hours) | Percent Dissolved |
|---|---|
| 1 | 28.5 |
| 2 | 42.7 |
| 4 | 61.5 |
| 8 | 83.3 |
| 12 | 94.5 |
| 16 | 99.7 |
| 24 | 103.2 |

**Example 3: Bioequivalence study.**

Bioequivalence was studied according to accepted guidelines (see Food and Drug Administration, Guidance for Industry: Bioavailability and Bioequivalence Studies for Orally Administered Drug Products - General Considerations, Center for Drug Evaluation and Research, March 2003; see also Food and Drug Administration, Statistical Approaches to Establishing Bioequivalence, Center for Drug Evaluation and Research, January 2001). The study compared a single dose of 30 mg phenylephrine in a tablet according to the invention to two 10 mg phenylephrine immediate release tablets (Sudafed PE™ Nasal Decongestant Tablets 10 mg) dosed four hours apart. Twenty-four healthy volunteers were enrolled in the study. On study day 1, subjects were dosed at random with a single oral dose of either the test tablet or two doses of the comparison tablet. Following a three day washout period, subjects returned and were dosed with the alternative treatment per randomization. Drug administration was assisted with 240 mL of ambient temperature water.

During each study period, 29 blood samples (6 mL each) were collected from each subject at the following time points: within one hour prior to dosing (0 hour) and after dose administration at 0.25, 0.5, 0.75, 1, 1.25, 1.5, 1.75, 2, 3, 4, 4.25, 4.5, 4.75, 5, 5.25, 5.5, 5.75, 6, 7, 8, 10, 12, 14, 16, 18, 20, 22, and 24 hours. Overall, phenylephrine was well tolerated as either a single, oral dose of a 30 mg 8 hour extended release tablet or as two 10 mg immediate release tablets consecutively dosed four (4) hours apart when administered under fasting conditions. Figures 2 and 3 present the results graphically. A summary of the statistical analysis is presented in Tables 2-4.

### Table 2 - Summary of Ln-Transformed Pharmacokinetic Parameters

| Phenylephrine | Ln-Transformed $C_{max}$ | Ln-Transformed $AUC_{0-t}$ | Ln-Transformed $AUC_{0-\infty}$ |
|---|---|---|---|
| Test Product Geometric Mean | 82.83 | 329.98 | 392.00 |
| Reference Product Geometric Mean | 89.28 | 356.81 | 450.68 |
| % Ratio | 92.77 | 92.48 | 86.98 |
| 90% Confidence Intervals | (86.22, 99.82) | (86.6, 98.76) | (80.84, 93.59) |

### Table 3 - Summary of Pharmacokinetic Parameters

| Phenylephrine | $C_{max}$ | $AUC_{0-t}$ | $AUC_{0-\infty}$ |
|---|---|---|---|
| Test Product Least Squares Mean | 86.26 | 346.94 | 412.34 |
| Reference Product Least Squares Mean | 92.04 | 363.35 | 469.51 |
| % Ratio | 93.72 | 95.48 | 87.82 |
| 90% Confidence Intervals | (87.22, 100.22) | (89.79, 101.17) | (80.29, 95.35) |

### Table 4 - Summary of Pharmacokinetic Parameters (continued)

| Phenylephrine | $T_{max}$* | $k_e$ | $t_{1/2}$ | $C_t$ |
|---|---|---|---|---|
| Test Product Least Squares Mean | NA | 0.3500 | 2.09 | 5.80 |

(continued)

| Phenylephrine | $T_{max}$* | $k_e$ | $t_{1/2}$ | $C_t$ |
|---|---|---|---|---|
| Reference Product Least Squares Mean | NA | 0.4802 | 1.60 | 5.88 |
| % Ratio | NA | 72.89 | 129.99 | 98.67 |
| 90% Confidence Intervals | NA | (60.05, 85.72) | (109.4, 150.58) | (97.55, 99.79) |
| *The p-value for $T_{max}$ (<0.0001) is based on the Wilcoxon Rank Sum Test | | | | |

The 90% confidence intervals about the ratio of the test geometric mean to the reference geometric mean are within the 80% and 125% limits for the pharmacokinetic parameters $C_{max}$, $AUC_{0-t}$, and $AUC_\infty$ of the In-transformed data.

Relevant parameters are understood as follows:

| | |
|---|---|
| $C_{max}$ | Peak drug concentration obtained directly from the data without interpolation. |
| $T_{max}$ | Time to peak drug concentration obtained directly from the data without interpolation. |
| $C_t$ | The last measured plasma concentration; the last concentration above the lower LOQ following a dose. |
| $\lambda_z(k_{elim})$ | Terminal or elimination rate constant determined from the slope of the drug concentration-time curve using linear regression on terminal data points of the curve. |
| $AUC_{0-t}$ | Area under the concentration-time curve from zero to time t, calculated by the trapezoidal rule, where t is the last time point with measurable concentration. |
| $AUC_{0-\infty}$ | Area under the concentration-time curve from time zero to time infinity, calculated by the following: |

$$AUC_{0-\infty} = AUC_{0-t} + C_t/\lambda_z$$

Where $C_t$ is the last measurable drug concentration and $\lambda_z$ is the terminal or elimination rate constant.

$t_{1/2}$       Terminal or elimination half-life of the drug, calculated from the equation:

$$t_{1/2} = \ln(2)/\lambda_z$$

**Example 4 - Dual release phenylephrine sustained release tablet.**

The following example provides a pharmaceutical formulation comprising phenylephrine in two parts, the first part comprising an immediate release formulation and the second part comprising between about 18 - 22 mg phenylephrine in a zero order, or near zero order, sustained release formulation. The immediate released layer could be as active coating or an immediate released layer of a two or three layered tablet. The zero or near zero order sustained release portion could be as core tablet or as a layer of a two or three layered tablet.

Accordingly, the composition can comprise the following sustained release portion and immediate release portions.

**Sustained released portion:**

| Component | Weight Percent Ranges |
|---|---|
| Phenylephrine Hydrochloride | 1- 50 |
| Microcrystalline Cellulose NF | 0 - 60 |
| Carboxymethylcellulose Sodium or Calcium salt | 10 - 60 |
| Hydroxypropylcellulose | 20 - 40 |
| Silicon Dioxide Colloidal | 0 - 2 |
| Magnesium Stearate | 0.2- 2.0 |

**Three part Immediate released coating:**
**Part A:** Seal Coat:

| Component | Target Weight (g/tablet) | Target Weight Percentages in solution (w/w) |
|---|---|---|
| Water USP | 0.00 | 0.00 |
| Polyethylene Glycol 3350 | 1.67 | 2.28% |
| Methocel E-5 Premium LV | 8.33 | 11.36% |
| Totals | 10.00 | 13.64% |

**Part B:** Active Coat. (Assuming 10 mg PE in coating)

| Component | Target Weight (g/tablet) | Target Weight Percentages in solution (w/w) |
|---|---|---|
| Water USP | 0.00 | 0.00 |
| Polyethylene Glycol 3350 | 3.65 | 1.35% |
| Methocel E-5 Premium LV | 14.60 | 5.38% |
| Loratadine | 5.00 | 1.84% |
| Opaspray Blue K-1-4108* | 8.20 | 3.02% |
| Phenylephrine HCl 150 mesh | 10.00 | 3.69% |
| Totals | 41.45 | 15.28% |

Additional information for Active Coat (B): Potential Ranges for PE

| Component | Target Weight (g/tablet) | Target Weight Percentages in solution (w/w) |
|---|---|---|
| Phenylephrine HCl 150 mesh | 8.00 | 3.10% |
| Phenylephrine HCl 150 mesh | 12.00 | 4.22% |

**Part C:** Seal Coat:

| Component | Target Weight (g/tablet) | Target Weight Percentages in solution (w/w) |
|---|---|---|
| Water USP | 0.00 | 0.00 |
| Polyethylene Glycol 400 | 0.75 | 2.27% |
| Methocel E-5 Premium LV | 3.75 | 11.37% |
| Totals | 4.50 | 13.64% |

The results presented in the Examples and Figures are non-limiting. From the above description, one can ascertain the essential characteristics of the present invention and, without departing from the spirit and scope thereof, can make various changes and modifications of the invention to adapt it to various uses and conditions.

**Claims**

1. Pharmaceutical composition formulated for sustained release of therapeutically effective phenylephrine dose for at least eight hours after a single dose is administered to a human subject, the composition comprising phenylephrine within a polymer matrix, wherein the composition exhibits a mean AUC and/or $C_{max}$ in the human subject equivalent to 80% to 125% of the AUC and/or $C_{max}$ obtained when two doses of a standard immediate release formulation comprising 10 mg of phenylephrine is administered.

2. Pharmaceutical composition formulated for sustained release of therapeutically effective phenylephrine dose for at least twelve hours after a single dose is administered to a human subject, the composition comprising phenylephrine within a sustained release polymer matrix, wherein the composition exhibits a mean AUC and/or $C_{max}$ in the human subject equivalent to 80% to 125% of the AUC and/or $C_{max}$ obtained when three doses of a standard immediate release formulation comprising 10 mg of phenylephrine is administered.

3. Pharmaceutical composition formulated for sustained release of therapeutically effective phenylephrine dose for at least twenty four hours after a single dose is administered to a human subject, the composition comprising phenylephrine within a polymer matrix, wherein the composition exhibits a mean AUC and/or $C_{max}$ in the human subject equivalent to 80% to 125% of the AUC and/or $C_{max}$ obtained when six doses of a standard immediate release formulation comprising 10 mg of phenylephrine is administered.

4. Pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by two doses of a pharmaceutical composition containing 10 mg of phenylephrine and no sustained release polymer matrix.

5. Pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by three doses of a pharmaceutical composition containing 10 mg of phenylephrine and no sustained release polymer matrix.

6. Pharmaceutical composition comprising phenylephrine or a pharmaceutically acceptable salt thereof in a sustained release polymer matrix, wherein said composition when administered once to a subject exhibits a mean AUC and/or $C_{max}$ which is at least 80% of the mean AUC and/or $C_{max}$ exhibited by six doses of a pharmaceutical composition containing 10 mg of phenylephrine and no sustained release polymer matrix.

7. Pharmaceutical composition of any of claims 1 to 6 wherein the sustained release polymer matrix comprises hydroxypropyl methylcellulose.

8. Pharmaceutical composition comprising phenylephrine or a pharmaceutically acceptable salt thereof, wherein the composition exhibits an sustained release of phenylephrine from the composition when contacted with USP simulated intestinal fluid.

9. Pharmaceutical composition of claim 8 that provides a sustained release for a period of at least 8 hours such that a determination can be made that less than the total amount of phenylephrine has been dissolved in the USP simulated intestinal fluid over said time period.

10. Pharmaceutical composition comprising phenylephrine or a pharmaceutically acceptable salt thereof, wherein the composition exhibits an sustained release of phenylephrine from the composition when contacted with USP simulated intestinal fluid, such that no more than 30 percent of the phenylephrine or pharmaceutically acceptable salt thereof is released from the pharmaceutical composition within one hour, no more than 60 percent of the phenylephrine or pharmaceutically acceptable salt thereof is released from the pharmaceutical composition within four hours, no more than 90 percent of the phenylephrine or pharmaceutically acceptable salt thereof is released from the pharmaceutical composition within about 8 hours, and at least 99 percent of the phenylephrine or pharmaceutically acceptable salt thereof is released from the pharmaceutical composition within about 24 hours.

11. Composition of claim 10 comprising an additional therapeutic agent.

12. Composition of claim 11 wherein the additional therapeutic agent is chosen from the group consisting of an antihistamine, an analgesic and combinations thereof.

13. Composition of claim 12 wherein the antihistamine is chosen from the group consisting of diphenhydramine, chlorpheniramine, tripelennamine, promethazine, clemastine, doxylamine, astemizole, terfenadine, loratadine, cimetadine, famotidine, nizatidine, ranitidine, cromolyn and combinations thereof.

FIGURE 1
Dissolution Profile of a Tablet

FIGURE 2
Mean Plasma Concentration (0 – 24 hours)

**N=24**

FIGURE 3
Mean Plasma Concentration (0 – 24 hours) Semi-Logarithmic Scale

N=24

Europäisches
Patentamt

European
Patent Office

Office européen
des brevets

# EUROPEAN SEARCH REPORT

Application Number

EP 10 17 3302

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/095288 A1 (HONEA DICKEY [US]) 5 May 2005 (2005-05-05) * paragraph [0003] * * paragraph [0022] - paragraph [0026] * * paragraph [0038] - paragraph [0063] * * examples 7,10 * ----- | 1-13 | INV. A61K9/22 A61K9/36 A61K31/137 |
| X | US 2005/152967 A1 (TENGLER MARK [US] ET AL) 14 July 2005 (2005-07-14) * claims 1-8 * * paragraph [0045]; figures 1-3 * ----- | 1 | |
| X | US 4 294 820 A (KEITH ALEC D ET AL) 13 October 1981 (1981-10-13) * column 2, line 4 - line 7 * * claim 1 * ----- | 1 | |
| X | US 3 558 768 A (KLIPPEL KENNETH R) 26 January 1971 (1971-01-26) * claim 1 * * example 3 * ----- | 1,7-9 | |
| A | HAMDANI JAMILA ET AL: "Development and evaluation of prolonged release pellets obtained by the melt pelletization process" INTERNATIONAL JOURNAL OF PHARMACEUTICS (KIDLINGTON), vol. 245, no. 1-2, 1 October 2002 (2002-10-01), pages 167-177, XP002484422 ISSN: 0378-5173 * abstract * * paragraph [02.4] * ----- | 1,7 | TECHNICAL FIELDS SEARCHED (IPC)  A61K |

The present search report has been drawn up for all claims

| Place of search | Date of completion of the search | Examiner |
|---|---|---|
| Munich | 30 September 2010 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 17 3302

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2010

| Patent document cited in search report | | Publication date | Patent family member(s) | | Publication date |
|---|---|---|---|---|---|
| US 2005095288 | A1 | 05-05-2005 | NONE | | |
| US 2005152967 | A1 | 14-07-2005 | NONE | | |
| US 4294820 | A | 13-10-1981 | US | 4291015 A | 22-09-1981 |
| | | | US | 4289749 A | 15-09-1981 |
| | | | US | 4292301 A | 29-09-1981 |
| | | | US | 4292302 A | 29-09-1981 |
| | | | US | 4292303 A | 29-09-1981 |
| | | | US | 4321252 A | 23-03-1982 |
| | | | US | 4470962 A | 11-09-1984 |
| | | | US | 4472372 A | 18-09-1984 |
| | | | US | 4466953 A | 21-08-1984 |
| | | | US | 4492685 A | 08-01-1985 |
| US 3558768 | A | 26-01-1971 | NONE | | |

EPO FORM P0459

For more details about this annex : see Official Journal of the European Patent Office, No. 12/82

**REFERENCES CITED IN THE DESCRIPTION**

**Patent documents cited in the description**

- US 4792452 A
- AU 5676186 B
- JP 2005060294 A
- US 4282233 A
- BE 647043
- US 3326924 A

- US 3419565 A
- US 3878217 A
- US 4525358 A
- US 4219559 A
- US 4369184 A

**Non-patent literature cited in the description**

- **Gennaro et al.** Remington's Pharmaceutical Sciences. Mack Publishing Company, 1995
- Food and Drug Administration. Guidance for Industry: Bioavailability and Bioequivalence Studies for Orally Administered Drug Products - General Considerations. Center for Drug Evaluation and Research, March 2003

- Food and Drug Administration. Statistical Approaches to Establishing Bioequivalence. Center for Drug Evaluation and Research, January 2001